(19) Europäisches Patentamt
European Patent Office
Office européen des brevets

(11) Publication number: **0 105 590**
**B1**

(12) **EUROPEAN PATENT SPECIFICATION**

(45) Date of publication of patent specification: **30.12.86**

(21) Application number: **83304850.7**

(22) Date of filing: **23.08.83**

(51) Int. Cl.⁴: **C 07 D 231/40,**
C 07 D 213/75,
C 07 D 239/42,
C 07 D 261/14,
C 07 D 275/02,
C 07 D 271/06,
C 07 D 233/88,
C 07 D 251/42,
C 07 D 263/48,
C 07 D 277/48, C 07 D 285/08

(54) **Process for the production of guanidinoheterocyclic derivatives.**

(30) Priority: **07.09.82 GB 8225509**

(43) Date of publication of application:
**18.04.84 Bulletin 84/16**

(45) Publication of the grant of the patent:
**30.12.86 Bulletin 86/52**

(84) Designated Contracting States:
**AT BE CH DE FR GB IT LI LU NL SE**

(56) References cited:
**EP-A-0 060 094**
**EP-A-0 060 697**

(73) Proprietor: **IMPERIAL CHEMICAL INDUSTRIES PLC**
**Imperial Chemical House Millbank**
**London SW1P 3JF (GB)**

(72) Inventor: **Hales, Neil James**
**12 Melksham Close**
**Macclesfield Cheshire (GB)**
Inventor: **Mant, Derrick Michael**
**115 Moss Lane**
**Bramhall Cheshire (GB)**

(74) Representative: **Brown, Ivor James Stewart et al**
**Imperial Chemical Industries PLC Legal Department: Patents PO Box 6**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the manufacture of guanidine derivatives.

In European Patent Publications Nos. 60094 and 60697 there are described and claimed certain guanidinoheterocyclic derivatives which are histamine H—2 receptor antagonists. It has now been discovered that these compounds may be prepared using a novel method of forming the guanidine residue.

According to the invention there is provided a process for the manufacture of a guanidine derivative of the formula I:—

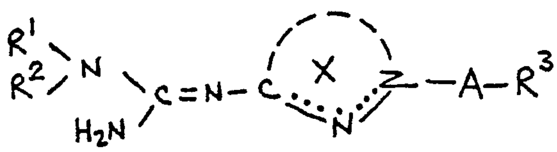

I

in which $R^1$ and $R^2$, which may be the same or different, are hydrogen or branched or unbranched 1—10C alkyl, 3—8C cycloalkyl or 4—14C cycloalkylalkyl, each alkyl, cycloalkyl or cycloalkylalkyl being optionally substituted by one or more halogens selected from fluorine, chlorine and bromine, provided that at least one of $R^1$ and $R^2$ is halogen-substituted alkyl, cycloalkyl or cycloalkylalkyl and provided there is no halogen substituent on the carbon of the alkyl, cycloalkyl or cycloalkylalkyl which is directly attached to the nitrogen, or —$R^2$ is hydrogen and —$R^1$ is of the formula II:—

$$R^4\text{–E–W–} \qquad \text{II}$$

in which W is an unbranched 2—6C alkylene chain which is optionally substituted by one or two 1—4C alkyls, E is oxygen, sulphur, sulphinyl, sulphonyl or, when $R^4$ is alkyl, of the formula $NR^5$ in which $R^5$ is 1—6C alkyl, $R^4$ is hydrogen or an unbranched 1—6C alkyl which is optionally substituted by one or two 1—4C alkyls, or $R^4$ and $R^5$ are joined to form, together with the nitorgen to which they are attached, a pyrrolidine, piperidine, morpholine, piperazine or N-methylpiperazine ring;

in ring X the dotted lined is a double bond on one side of the nitrogen and Z is carbon or nitrogen such that ring X is a 5- or 6-membered aromatic heterocyclic ring which contains at least one nitrogen and may optionally contain one or two additional hetero atoms selected from oxygen, nitrogen and sulphur, which heterocyclic ring may, where possible, carry one or two optional substituents, the optional substitutents on ring X being selected from fluorine, chlorine, bromine, 1—6C alkyl, 1—6C alkoxy, trifluoromethyl and hydroxy; A is phenylene or 5—7C cycloalkylene or a 1—8C alkylene chain which is optionally substituted by one or two 1—3C alkyls and into which is optionally inserted, as part of the backbone of the chain, one or two groups selected from oxygen, sulphur, NH, 1—6C N-alkyl, *cis* and *trans* vinylene, ethynylene, phenylene and 5—7C cycloalkylene, provided that the shortest link between ring X and $R^3$ is of at least 3 atoms, provided that when an optional insertion is made in chain A which results in the inserted group being directly attached to $R^3$ the inserted group is other than oxygen, sulphur, NH or N-alkyl and provided that no two insertions selected from oxygen, sulphur, NH and N-alkyl are directly attached one to the other;

—$R^3$ is of the formula III:—

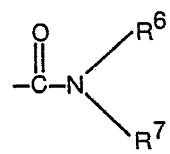

III

in which $R^6$ is hydrogen, hydroxy, 1—6C alkanoylamino, 1—6C alkyl, 3—8C cycloalkyl, 4—12C cycloalkylalkyl, 2—6C alkenyl, 2—6C alkynyl, 1—6C alkoxy, 1—6C hdyroxyalkyl, 2—10C alkoxyalkyl, 2—10C alkylthioalkyl, 3—12C dialkylaminoalkyl, 2—8C alkanoylaminoalkyl, 8—14C aroylaminoalkyl, 3—10C alkoxycarbonylalkyl, 2—8C carbamoylalkyl, 6—10C aryl, 7—11C arylalkyl, heteroaryl or heteroarylalkyl, wherein the heteroaryl part is a heterocyclic aromatic ring containing one, two or three hetero atoms selected from oxygen, nitrogen and sulphur, wherein the alkyl part of the heteroarylalkyl radical is 1—6C and wherein, when $R^6$ is or contains an aryl or heteroaryl ring, that ring is optionally substituted by one or two groups selected from fluorine, chlorine, bromine and iodine 1—6C alkyl, 1—6C alkoxy, 1—6C alkylthio, 2—6C dialkylamino, 2—6C alkanoyl, trifluoromethyl and hydroxy; $R^7$ is hydrogen or $R^6$ and $R^7$ are joined to form, together with the nitrogen to which they are attached, a 5-, 6- or 7-membered saturated ring which optionally contains a double bond or an additional oxygen or 1—6C N-alkyl;

or —$R^3$ is of the formula IV:—

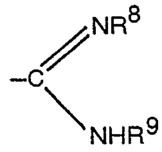

IV

in which $R^8$ and $R^9$ are joined to form, together with the N—C = N chain to which they are attached, a monocyclic or bicyclic heterocyclic ring system composed of 5- and/or 6-membered rings, which ring system may be partially unsaturated or fully unsaturated, which ring system may optionally include additional hetero atoms selected from oxygen, nitrogen and sulphur and which ring system may, where possible, carry one, two or three optional substituents selected from fluorine, chlorine, bromine, 1—6C alkyl, 1—6C alkoxy, 1—6C alkylthio, trifluoromethyl, hydroxy, 6—10C aryl, 7—11C arylalkyl, carboxy, 2—6C carboxyalkyl, 2—6C alkoxycarbonyl, 3—10C alkoxycarbonylalkyl, 1—6C hydroxyalkyl, heteroaryl-(1—6C)alkyl, furyl, thienyl, pyrrolyl, thiazolyl, oxazolyl, imidazolyl, thiadiazolyl, oxadiazolyl, triazolyl, pyrazolyl and pyrimidyl;
and wherein when $R^8$ and $R^9$, when joined, is substituted by heteroarylalkyl, that heteroaryl is a 5- or 6-membered heterocyclic ring which contains 1, 2, 3 or 4 heteroatoms selected from oxygen, nitrogen and sulphur, such ring being optionally substituted by one or two methyls;
or $R^8$ and $R^9$ are joined to form, together with the N—C = N chain to which they are attached, a ring of the formula V:—

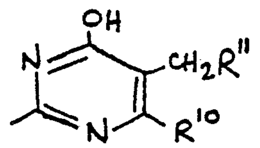

V

in which $R^{10}$ is hydrogen or 1—4C alkyl and $R^{11}$ is furan-2-yl or thien-2-yl substituted in the 5-position, phenyl substituted in the 3- or 4-position, pyrid-3-yl substituted in the 5- or 6-position, pyrid-4-yl substituted in 2-position, or pyrid-2-yl substituted in the 4- or 5-position, the substituent on $R^{11}$ being of the formula VI:—

$$R^{12}R^{13}N-(CH_2)_m- \qquad VI$$

in which $R^{12}$ and $R^{13}$ are 1—4C alkyls or $R^{12}$ and $R^{13}$ are joined to form, together with the nitrogen to which they are attached, a pyrrolidine, piperidine or morpholine ring and m is 1 to 4;
and the pharmaceutically-acceptable acid-addition salts thereof, characterised by reaction of an isourea of the formula VII or VIII:—

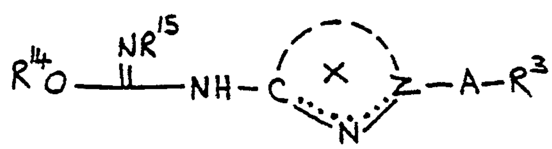

VII

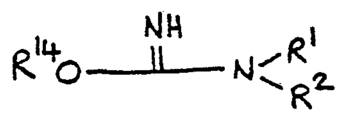

VIII

in which $R^{14}$ is optionally-substituted aryl and $R^{15}$ is hydrogen or one of the values given above for $R^1$ other than hydrogen, with ammonia or an amine which is of the formula $R^1R^2NH$, or with an amine of the formula X:—

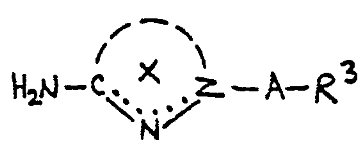

X

respectively;
whereafter, when the compound of the formula I is obtained in the form of the free base and an acid-addition salt is required, the compound of the formula I in the free-base form is reacted with an acid which affords a pharmaceutically acceptable anion.

Since in the compound of the formula I the guanidine residue carries three different substituents, the appropriate isourea for use in the process may have the formula VII or VIII:—

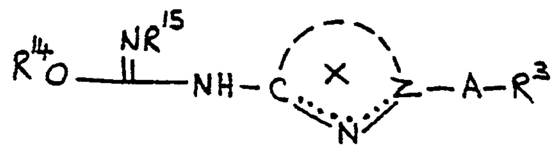

VII

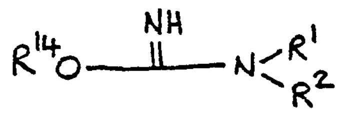

VIII

in which $R^{14}$ is optionally substituted aryl and $R^{15}$ is hydrogen or one of the values given above for $R^1$ other than hydrogen.

When the isourea has formula VII in which $R^{15}$ is hydrogen, the appropriate amine for use in the process is of the formula $R^1R^2NH$. When the isourea has formula VII in which $R^{15}$ has one of the values given above for $R^1$ other than hydrogen, the appropriate amine for use in the process is ammonia. When the isourea has formula VIII, the appropriate amine for use in the process is of the formula X:—

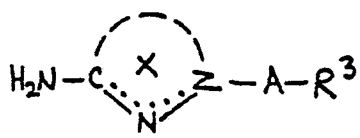

X

A particular value for $R^{14}$ is phenyl or naphthyl which is optionally substituted by one or two, substituents selected from halogen (e.g. F, Cl, Br, I), nitro (one only), 1—6C alkyl (e.g. methyl), 1—6C alkoxy (e.g. methoxy) and phenoxy.

A particular value for $R^1$ or $R^2$ when it is halogen-substituted is 2,2,2-trifluoroethyl, 2,2,2-trichloroethyl, 2-chloro-2,2-difluoroethyl, 2,2-dichloro-2-fluoroethyl, 2-bromo-2,2-difluoroethyl, 2,2-dibromo-2-fluoroethyl, 2-fluoroethyl, 2-chloroethyl, 2,2-difluoroethyl, 2,2-dichloroethyl, 2-chloro-2-fluoroethyl, 2-bromo-2-fluoroethyl, 2,2,3,3-tetrafluoropropyl, 2,2,3,3,3-pentafluoropropyl, 1,1,1,3,3,3-hexafluoroisopropyl, 1,3-dichloro-1,1,3,3-tetrafluoroisopropyl, 1-chloro-1,1,3,3,3-pentafluoroisopropyl, 1,3-difluoroisopropyl, 2,2,3,3,4,4,4-heptafluorobutyl, 2,2,3,3-tetrafluorocyclopropyl, 2-chloro-2,3,3-trifluorocyclopropyl, 2,2-difluorocyclopropyl, 2-chloro-3,3-difluorocyclopropyl, 2,2,3,3,4,4-hexafluorocyclobutyl, 2-chloro-2,3,3,4,4-pentafluorocyclobutyl, (1,2,2,3,3-pentafluorocyclopropyl)methyl, (2-chloro-1,2,3,3-tetrafluorocyclopropyl)methyl, (1,2,2,3,3,4,4-heptafluorocyclobutyl)methyl or (2-chloro-1,2,3,3,4,4-hexafluorocyclobutyl)-methyl.

A particular value for $R^1$ or $R^2$ when it is non-halogen substituted is methyl, ethyl, propyl, isopropyl, butyl, cyclopropyl, cyclobutyl, cyclopropylmethyl or cyclobutylmethyl.

A particular value for the optional substituent on W is methyl.

A particular value for $R^4$ is hydrogen or methyl.

A particular value for $R^5$ is methyl.

A particular value for the radical of the formula II is 2-methoxyethyl, 2-hdyroxyethyl, 2-methylthioethyl or 2-dimethylaminoethyl.

A particular value for ring X is an oxazole, thiazole, imidazole, 1,2,4-thiadiazole, 1,2,4-oxadiazole, 1,2,3-triazole, 1,2,4-triazole, pyrazole, pyrazine, pyridine, pyrimidine or 1,3,5-triazine ring, each being optionally substituted, where possible, by one or two substituents selected from fluorine, chlorine, bromine, methyl, methoxy, trifluoromethyl and hydroxy.

A particular value for —A— is phenylene, cyclopentylene, cyclohexylene, trimethylene, tetramethylene, pentamethylene, thioethylene, thiotrimethylene, thiotetramethylene, thiopentamethylene, oxyethylene, oxytrimethylene, oxytetramethylene, methylenethiomethylene, methylenethioethylene, methylenethiopropylene, methyleneoxymethylene, methyleneoxyethylene, ethyleneoxyethylene, oxy-2-methylethylene, thiopropylenethiomethylene, oxyethyleneoxymethylene, iminoethylene, iminopropylene, vinylenepropylene, oxymethylenevinylene, 1,3-phenylene, 1,3-cyclopentylene, methylene-1,4-phenylene, ethyleneoxymethylene-1,4-phenylene, oxy-1,3-phenylenemethylene or thiomethyleneethynylenemethylene. These values for —A— are written reading from left to right in formula I such that the first named part of the radical is attached to ring X and the last named part of the radical is attached to $R^3$. Thus, for example, when —A— is thiomethyleneethynylenemethylene, the compound of the formula I contains the part structure IX:—

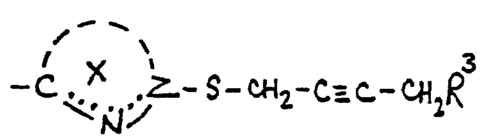

IX

A particular value for $R^6$ is hydrogen, hydroxy, acetylamino, methyl, cyclohexyl, cyclohexylmethyl, allyl, propargyl, methoxy, 2-hydroxyethyl, 2-methoxyethyl, 2-methylthioethyl, 2-dimethylaminoethyl, 2-

acetylaminoethyl, 2-benzoylaminoethyl, methoxycarbonylmethyl, 2-carbamoylpropyl, phenyl, benzyl, heteroaryl or heteroarylmethyl, in the latter two of which the heteroaryl part is furan, thiophene, pyrrole, thiazole, oxazole, imidazole, thiadiazole, oxadiazole, triazole, pyrazole, pyridine or pyrimidine, and wherein when $R^6$ is or contains phenyl or heteroaryl, that ring is optionally substituted by one or two groups selected from fluorine, chlorine, bromine, iodine, methyl, methoxy, methylthio, dimethylamino, acetyl, trifluoromethyl and hydroxy.

A particular value for the ring formed when $R^6$ and $R^7$ are joined is pyrrolidone, piperidine, morpholine or N-methylpiperazine.

A particular value for the ring system formed when $R^8$ and $R^9$ are joined is an imidazole, imidazoline, triazole, pyrimidine, oxadiazole, thiadiazole, 1,3,5-triazine, 1,2,4-triazine, benzimidazole, quinazoline or purine (linked through the 2- or 8- position) ring system, each of which ring systems may, where possible, carry one, two or three optional substituents selected from fluorine, chlorine, bromine, methyl, ethyl, propyl, butyl, methoxy, methylthio, trifluoromethyl, hydroxy, phenyl, benzyl, carboxymethyl, methoxycarbonyl, methoxycarbonylmethyl, hydroxymethyl, furyl, thienyl, pyrrolyl thiazolyl, oxazolyl, imidazolyl, thiadiazolyl, oxadiazolyl, triazolyl, pyrazolyl and pyrimidyl, and heteroarylmethyl and 2-heteroarylethyl in which the heteroaryl part is furyl, thienyl, pyrrolyl, thiazolyl, oxazolyl, imidazolyl, thiadiazolyl, ozadiazolyl, triazolyl, pyrazolyl, pyridyl or pyrimidyl, each optionally substituted by one or two methyls.

A further particular value for the ring formed when $R^8$ and $R^9$ are joined is a ring of the formula V given above in which $R^{10}$ is hydrogen or methyl and $R^{11}$ is furan-2-yl or thien-2-yl substituted in the 5-position, phenyl substituted in the 3- or 4-position, pyrid-3-yl substituted in the 5- or 6-position, pyrid-4-yl substituted in the 2-position or pyrid-2-yl substituted in the 4- or 5-position, the substituent on $R^{11}$ being a radical of the formula VI given above in which $R^{12}$ and $R^{13}$ are methyl or are joined to form, together with the nitrogen to which they are attached, pyrrolidine, piperidine or morpholine and m is 1 to 4, preferably 1.

A preferred compound which may be manufactured by the process is that of the formula I in which $R^1$ is 2,2,2-trifluoroethyl, $R^2$ is hydrogen, ring X is a pyrazole ring, A is tetramethylene and $R^3$ is of the formula III in which $R^6$ and $R^7$ are hydrogen.

The process of the invention may be carried out in a diluent or solvent such as acetonitrile, and may be accelerated or completed by the application of heat, for example by heating to the boiling point of the diluent or solvent.

The starting material of the formula VII in which $R^{15}$ is hydrogen may be prepared by reaction of a compound of the formula $R^{14}OCN$ with a compound of the formula X:—

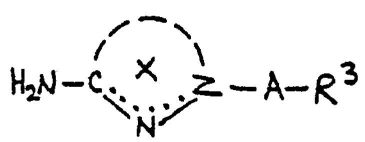

X

for example as described in Example I. The compound of the formula X may be prepared as described in European Patent Publications 60094 and 60697.

The starting material of the formula VII in which $R^{15}$ is other than hydrogen may be prepared by reaction of a compound of the formula XI:—

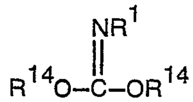

XI

[itself prepared by N-alkylation of a compound of the formula $(R^{14}O)_2C = NH$] with a compound of the formula X.

The starting material of the formula VIII may be prepared by reaction of a compound of the formula $R^{14}OCN$ with an amine of the formula $R^1R^2NH$, for example as described in Example 2.

The process is illustrated, but not limited, by the following Examples in which the temperatures are in degrees Centigrade and the following contractions are used:—

| | |
|---|---|
| MeOH | = methanol |
| EtOAc | = ethyl acetate |
| DMF | = dimethyl formamide |
| EtOH | = ethanol |

Example 1

A mixture of 5-(3-[2-(4-nitrophenyl)isoureido]pyrazol-1-yl)valeramide hydrochloride (0.16 g.), triethylamine (0.06 ml.), 2,2,2-trifluoroethylamine (0.2 g.) and acetonitrile (5 ml.) was heated under reflux overnight. The solvent was evaporated and the residue was purified by medium pressure liquid chromatography on silica-gel using first $CHCl_3$/MeOH/aqueous ammonia (s.g. 0.88) 9:1:0.1 v/v/v and then

MeOH as eluant to give 0.09 g. (70%) of 5-(3-[2-(2,2,2-trifluoroethyl)guanidino]pyrazol-1-yl)valeramide, m.p. 130° on recrystallisation from EtOAc.

The starting material may be obtained as follows:—

Sodium hydride paste (6.16 g. of 61% w/w suspension in liquid paraffin) was added portionwise over 30 minutes to a solution of 3-nitropyrazole (17.4 g.) in dry DMF (150 ml.) with external ice cooling to maintain the temperature at 20—30°. The mixture was stirred for 45 minutes and to the almost clear solution was added 5-bromovaleronitrile (25 g.) over 30 minutes, at 25—30°, and the mixture was stirred for 4 hours. Water (450 ml.) and EtOAc (450 ml.) was added and the upper layer was separated, dried ($MgSO_4$) and evaporated *in vacuo* to an oil which was a mixture of 5-(3-nitropyrazol-1-yl)valeronitrile and 5-(5-nitropyrazol-1-yl)valeronitrile. The oil was divided into two 15 g. portions which were fractionated on a silica column (3.5 cm. diameter × 100 cm. long) eluted at 2 atmospheres by EtOAc/60—80° petroleum ether (3:7 v/v). The 1:5 isomer was eluted first followed by the 1:3 isomer. The 5-(3-nitropyrazol-1-yl)valeronitrile had m.p. 32—33°.

A solution of 5-(3-nitropyrazol-1-yl)valeronitrile (0.2 g.) in concentrated sulphuric acid (1 ml.) was kept at 20° for 19 hours. The mixture was diluted with water (4 ml.), basified to pH 10 with 10.8N sodium hydroxide and extracted with EtOAc (3 × 5 ml.). The extracts were dried ($MgSO_4$) and evaporated *in vacuo* to a white solid which was recrystallised from EtOH to give 5-(3-nitropyrazol-1-yl)valeramide, m.p. 129—131°.

A mixture of 5-(3-nitropyrazol-1-yl)valeramide (3.6 g.) and 3% w/w palladium on carbon (0.54 g.) was stirred in isopropanol (20 ml.) under an atmosphere of hydrogen. The temperature was kept below 40° by external ice cooling. After 4 hours no more hydrogen was absorbed. The mixture was filtered and the filtrate was evaporated *in vacuo* to give 5-(3-aminopyrazol-1-yl)valeramide as an oil which crystallised.

A mixture of 5-(3-aminopyrazol-1-yl)valeramide hydrochloride (prepared in EtOH with ethereal HCl followed by evaporation; 0.22 g.), 4-nitrophenylcyanate (*Chem. Ber.,* 1964, *97,* 3012; 0.16 g.) and acetonitrile (2 ml.) was stirred at room temperature overnight. A further 0.08 g. of 4-nitrophenylcyanate was then added and the mixture was stirred at room temperature overnight. A further 0.15 g. of 4-nitrophenylcyanate and 5 ml. of acetonitrile were then added and the mixture was stirred at room temperature overnight. The mixture was filtered. The retained solid was washed with acetonitrile and air-dried to give 0.16 g. (42%) of 5-(3-[2-(4-nitro phenyl)isoureido]pyrazol-1-yl)valeramide hydrochloride which was used without further purification.

Example 2

A mixture of 5-(3-aminopyrazol-1-yl)valeramide (1.0 g.), 1-(4-methylphenyl)-2-(2,2,2-trifluoroethyl)isourea (2.0 g.) and acetonitrile (10 ml.) was heated at 80° for 3 days. The mixture was twice treated with both 0.5 g. of 1-(4-methylphenyl)-2-(2,2,2-trifluoroethyl)isourea hydrochloride and 0.5 ml. of triethylamine and heated at 100° for 1 day. The solvent was evaporated and the mixture was purified by medium pressure liquid chromatography on silica-gel using $CHCl_3$/MeOH/aqueous ammonia (s.g. 0.88) 19:1:0.1 v/v/v as eluant to give 0.28 g. (17%) of 5-(3-[2-(2,2,2-trifluoroethyl)guanidino]pyrazol-1-yl)valeramide, identical with the sample prepared in Example 1.

The starting material may be prepared as follows:—

A mixture of 2,2,2-trifluoroethylamine hydrochloride (7.0 g.), 4-methyphenylcyanate (*Chem. Ber.,* 1964, *97,* 3012; 6.7 g.) and DMF (50 ml.) was stirred at room temperature for 4 days. The DMF was removed by distillation under reduced pressure. The residue was triturated with ether and filtered to give 10.8 g. of a solid. A portion of this solid (9.5 g.) was treated with an excess of saturated aqueous sodium bicarbonate and extracted with ether. The extract was dried ($MgSO_4$) and evaporated. The residual solid was triturated with light petroleum (b.p. 40—60°) and filtered to give 4.7 g. of 1-(4-methylphenyl)-2-(2,2,2-trifluoroethyl)isourea, m.p. 83—85°, 4.2 g. of which was treated with ethereal hydrogen chloride. The mixture was filtered to give 4.2 g. (39.5%) of the product as its hydrochloride salt, m.p. 180—182°.

**Claims**

1. A process for the manufacture of a guanidine derivative of the formula I:—

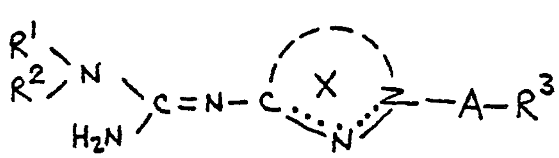

I

in which $R^1$ and $R^2$, which may be the same or different, are hydrogen or branched or unbranched 1—10C alkyl, 3—8C cycloalkyl or 4—14C cycloalkylalkyl, each alkyl, cycloalkyl or cycloalkylalkyl being optionally substituted by one or more halogens selected from fluorine, chlorine and bromine, provided that at least one of $R^1$ and $R^2$ is halogen-substituted alkyl, cycloalkyl or cycloalkylalkyl and provided there is no halogen substituent on the carbon of the alkyl, cycloalkyl or cycloalkylalkyl which is directly attached to the nitrogen, or $—R^2$ is hydrogen and $—R^1$ is of the formula

$R^4$–E–W– II

in which W is an unbranched 2—6C alkylene chain which is optionally substituted by one or two 1—4C alkyls, E is oxygen, sulphur, sulphinyl, sulphonyl or, when $R^4$ is alkyl, of the formula $NR^5$ in which $R^5$ is 1—6C alkyl, $R^4$ is hydrogen or an unbranched 1—6C alkyl which is optionally substituted by one or two 1—4C alkyls, or $R^4$ and $R^5$ are joined to form, together with the nitrogen to which they are attached, a pyrrolidine, piperidine, morpholine, piperazine or N-methylpiperazine ring;

in ring X the dotted line is a double bond on one side of the nitrogen and Z is carbon or nitrogen such that ring X is a 5- or 6-membered aromatic heterocyclic ring which contains at least one nitrogen and may optionally contain one or two additional hetero atoms selected from oxygen, nitrogen and sulphur, which heterocyclic ring may, where possible, carry one or two optional substituents, the optional substituents on ring X being selected from fluorine, chlorine, bromine, 1—6C alkyl, 1—6C alkoxy, trifluoromethyl and hydroxy;

A is phenylene or 5—7C cycloalkylene or a 1—8C alkylene chain which is optionally substituted by one or two 1—3C alkyls and into which is optionally inserted, as part of the backbone of the chain, one or two groups selected from oxygen, sulphur, NH, 1—6C N-alkyl, *cis* and *trans* vinylene, ethynylene, phenylene and 5—7C cycloalkylene, provided that the shortest link between ring X and $R^3$ is of at least 3 atoms, provided that when an optional insertion is made in chain A which results in the inserted group being directly attached to $R^3$ the inserted group is other than oxygen, sulphur, NH or N-alkyl and provided that no two insertions selected from oxygen, sulphur, NH and N-alkyl are directly attached one to the other;

—$R^3$ is of the formula III:—

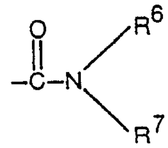

III

in which $R^6$ is hydrogen, hydroxy, 1—6C alkanoylamino, 1—6C alkyl, 3—8C cycloalkyl, 4—12C cycloalkylalkyl, 2—6C alkenyl, 2—6C alkynyl, 1—6C alkoxy, 1—6C hdyroxyalkyl, 2—10C alkoxyalkyl, 2—10C alkylthioalkyl, 3—12C dialkylaminoalkyl, 2—8C alkanoylaminoalkyl, 8—14C aroylaminoalkyl, 3—10C alkoxycarbonylalkyl, 2—8C carbamoylalkyl, 6—10C aryl, 7—11C arylalkyl, heteroaryl or heteroarylalkyl, wherein the heteroaryl part is a heterocyclic aromatic ring containing one, two or three hetero atoms selected from oxygen, nitrogen and sulphur, wherein the alkyl part of the heteroarylalkyl radical is 1—6C and wherein, when $R^6$ is or contains an aryl or heteroaryl ring, that ring is optionally substituted by one or two groups selected from fluorine, chlorine, bromine and iodine 1—6C alkyl, 1—6C alkoxy, 1—6C alkylthio, 2—6C dialkylamino, 2—6C alkanoyl, trifluoromethyl and hydroxy; $R^7$ is hydrogen or $R^6$ and $R^7$ are joined to form, together with the nitrogen to which they are attached, a 5-, 6- or 7-membered saturated ring which optionally contains a double bond or an additional oxygen or 1—6C N-alkyl;

or —$R^3$ is of the formula IV:—

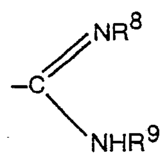

IV

in which $R^8$ and $R^9$ are joined to form, together with the N—C = N chain to which they are attached, a monocyclic or bicyclic heterocyclic ring system composed of 5- and/or 6-membered rings, which ring system may be partially unsaturated or fully unsaturated, which ring system may optionally include additional hetero atoms selected from oxygen, nitrogen and sulphur and which ring system may, where possible, carry one, two or three optional substituents selected from fluorine, chlorine, bromine, 1—6C alkyl, 1—6C alkoxy, 1—6C alkylthio, trifluoromethyl, hydroxy, 6—10C aryl, 7—11C arylalkyl, carboxy, 2—6C carboxyalkyl, 2—6C alkoxycarbonyl, 3—10C alkoxycarbonylalkyl, 1—6C hydroxyalkyl, heteroaryl-(1—6C)alkyl, furyl, thienyl, pyrrolyl, thiazolyl, oxazolyl, imidazolyl, thiadiazolyl, oxadiazolyl, triazolyl, pyrazolyl and pyrimidyl;

and wherein when $R^8$ and $R^9$, when joined, is substituted by heteroarylalkyl, that heteroaryl is a 5- or 6-membered heterocyclic ring which contains 1, 2, 3 or 4 heteroatoms selected from oxygen, nitrogen and sulphur, such ring being optionally substituted by one or two methyls;

or $R^8$ and $R^9$ are joined to form, together with the N—C = N chain to which they are attached, a ring of the formula V:—

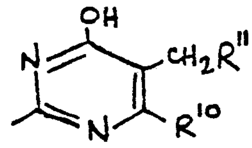

V

in which $R^{10}$ is hydrogen or 1—4C alkyl and $R^{11}$ is furan-2-yl or thien-2-yl substituted in the 5-position, phenyl substituted in the 3- or 4-position, pyrid-3-yl substituted in the 5- or 6-position, pyrid-4-yl substituted in the 2-position, or pyrid-2-yl substituted in the 4- or 5-position, the substituent on $R^{11}$ being of the formula

$$R^{12}R^{13}N-(CH_2)_m- \qquad VI$$

in which $R^{12}$ and $R^{13}$ are 1—4C alkyls or $R^{12}$ and $R^{13}$ are joined to form, together with the nitrogen to which they are attached, a pyrrolidine, piperidine or morpholine ring and m is 1 to 4;

and the pharmaceutically-acceptable acid-addition salts thereof, characterised by reaction of an isourea of the formula VII or VIII:—

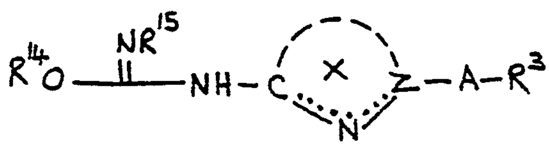

VII

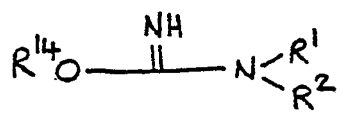

VIII

in which $R^{14}$ is optionally-substituted aryl and $R^{15}$ is hydrogen or one of the values given above for $R^1$ other than hydrogen, with ammonia or an amine which is of the formula $R^1R^2NH$, or with an amine of the formula X:—

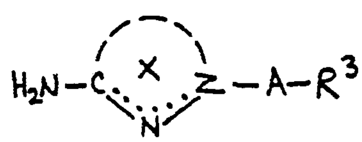

X

respectively;

whereafter when the process manufactures the compound of the formula I in the form of the free base and an acid-addition salt is required, the compound of the formula I in the free base form is reacted with an acid which affords a pharmaceutically-acceptable anion.

2. A process as claimed in claim 1 in which $R^{14}$ is phenyl or naphthyl which is optionally substituted by one or two substituents selected from halogen, nitro, (one only), 1—6C alkyl, 1—6C alkoxy, and phenoxy.

3. A process as claimed in claim 1 or 2 which is carried out in a diluent or solvent and which may be accelerated or completed by the application of heat.

4. A process as claimed in any of claims 1—3 in which, in the starting materials, $R^1$ is a 2,2,2-trifluoroethyl, $R^2$ is hydrogen, ring X is a pyrazole ring, A is tetramethylene and $R^3$ is of the formula III in which $R^6$ and $R^7$ are hydrogen.

**Patentansprüche**

1. Verfahren zur Herstellung eines Guanidin-Derivats der Formel I

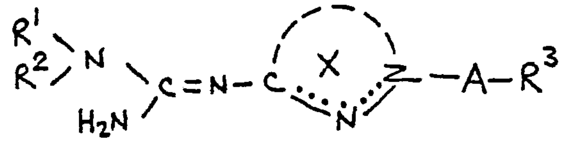

I

worin $R^1$ und $R^2$, welche gleich oder verschieden sein können, für Wasserstoff oder verzweigtes oder unverzweigtes 1—10C-Alkyl, 3—8C-Cycloalkyl oder 4—14C-Cycloalkylalkyl stehen, wobei jedes Alkyl, Cycloalkyl oder Cycloalkylalkyl gegebenenfalls durch ein oder mehrere aus Fluor, Chlor und Brom ausgewählte Halogene substituiert ist, mit der Maßgabe, daß mindestens eines der Symbole $R^1$ und $R^2$ für

halogensubstituiertes Alkyl, Cycloalkyl oder Cycloalkylalkyl steht, und mit der weiteren Maßgabe, daß sich kein Halogensubstituent an dem Kohlenstoff des Alkyls, Cycloalkyls oder Cycloalkylalkyls befindet, der direkt an den Stickstoff gebunden ist, oder

$R^2$ für Wasserstoff steht und $R^1$ die Formel

$$R^4\text{–}E\text{–}W\text{–} \qquad II$$

aufweist, worin W eine unverzweigte 2—6C-Alkylenkette bedeutet, die gegebenenfalls durch ein oder zwei 1—4C-Alkyle substituiert ist, E Sauerstoff, Schwefel, Sulfinyl oder Sulfonyl bedeutet oder, wenn $R^4$ Alkyl ist, die Formel $NR^5$ aufweist, worin $R^5$ 1—6C-Alkyl ist, und $R^4$ Wasserstoff oder ein unverzweigtes 1—6C-Alkyl bedeutet, das gegebenenfalls durch ein oder zwei 1—4C-Alkyle substituiert ist, oder $R^4$ und $R^5$ miteinander verbunden sind, so daß sie zusammen mit dem Stickstoff, an welchen sie geknüpft sind, einen Pyrrolidin-, Piperidin-, Morpholin, Piperazin- oder N-Methylpiperazin-Ring bilden;

im Ring X die gestrichelte Linie für eine Doppelbindung an einer Seite des Stickstoffs steht und Z für Kohlenstoff oder Stickstoff steht, derart, daß der Ring X ein 5- oder 6-gliedriger aromatischer heterocyclischer Ring ist, der mindestens einen Stickstoff enthält und der gegebenenfalls ein oder zwei zusätzliche Heteroatome enthalten kann, die aus Sauerstoff, Stickstoff und Schwefel ausgewählt sind, wobei der heterocyclische Ring, sofern möglich, einen oder zwei fakultative Substituenten tragen kann, die aus Fluor, Chlor, Brom, 1—6C-Alkyl, 1—6C-Alkoxy, Trifluoromethyl und Hydroxy ausgewählt sind;

A für Phenylen oder 5—7C-Cycloalkylen oder für eine 1—8C-Alkylenkette steht, die gegebenenfalls durch ein oder zwei 1—3C-Alkyle substituiert ist und in die gegebenenfalls als Teil des Gerüsts der Kette eine oder zwei Gruppen eingefügt sind, die aus Sauerstoff, Schwefel, NH, 1—6C-N-Alkyl, cis- und trans-Vinylen, Ethinylen, Phenylen und 5—7C-Cycloalkylen ausgewählt sind, mit der Maßgabe, daß die kürzeste Gruppierung zwischen dem Ring X und $R^3$ mindestens 3 Atome aufweist, mit der weiteren Maßgabe, daß, wenn eine fakultative Einfügung in der Kette A vorliegt, die zur Folge hat, daß die eingefügte Gruppe direkt an $R^3$ gebunden ist, die eingefügte Gruppe etwas anderes ist als Sauerstoff, Schwefel, NH oder N-Alkyl, und schließlich mit der Maßgabe, daß nicht zwei Einfügungen, die aus Sauerstoff, Schwefel, NH und N-Alkyl ausgewählt sind, direkt miteinander verbunden sind; und $R^3$ die Formel III

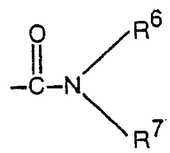

III

aufweist, worin $R^6$ Wasserstoff, Hydroxy, 1—6C-Alkanoylamino, 1—6C-Alkyl, 3—8C-Cycloalkyl, 4—12C-Cycloalkylalkyl, 2—6C-Alkenyl, 2—6C-Alkinyl, 1—6C-Alkoxy, 1—6C-Hydroxyalkyl, 2—10C-Alkoxyalkyl, 2—10C-Alkylthioalkyl, 3—12C-Dialkylaminoalkyl, 2—8C-Alkanoylaminoalkyl, 8—14C-Aroylaminoalkyl, 3—10C-Alkoxycarbonylalkyl, 2—8C-Carbamoylalkyl, 6—10C-Aryl, 7—11C-Arylalkyl, Heteroaryl oder Heteroarylalkyl bedeutet, worin der Heteroarylteil ein heterocyclischer aromatischer Ring ist, der ein, zwei oder drei Heteroatome enthält, die aus Sauerstoff, Stickstoff und Schwefel ausgewählt sind, worin weiterhin der Alkylteil des Heteroarylalkyl-Radikals 1 bis 6 Kohlenstoffatome aufweist, und worin schließlich, wenn $R^6$ ein Aryl- oder Heteroarylring ist oder einen solchen enthält, dieser Ring gegebenenfalls durch ein oder zwei Gruppen substituiert ist, die aus Fluor, Chlor, Brom, Jod, 1—6C-Alkyl, 1—6C-Alkoxy, 1—6C-Alkylthio, 2—6C-Dialkylamino, 2—6C-Alkanoyl, Trifluoromethyl und Hydroxy ausgewählt sind, und $R^7$ Wasserstoff bedeutet oder $R^6$ und $R^7$ miteinander verbunden sind, so daß sie zusammen mit dem Stickstoff, an welchen sie geknüpft sind, einen 5-, 6- oder 7-gliedrigen gesättigten Ring bilden, der gegebenenfalls eine Doppelbindung oder einen zusätzlichen Sauerstoff oder 1—6C-N-Alkyl enthält, oder $R^3$ die Formel IV

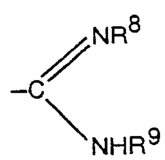

IV

aufweist, worin $R^8$ und $R^9$ miteinander verbunden sind, so daß sie zusammen mit der N—C = N -Kette, an welche sie geknüpft sind, ein monocyclsiches oder bicyclisches heterocyclisches Ringsystem bilden, das aus 5- und/oder 6-gleidrigen Ringen zusammengesetzt ist, welches Ringsystem teilweise ungesättigt oder vollständig ungesättigt sein kann, welches Ringsystem weiterhin gegebenenfalls zusätzliche Heteroatome enthalten kann, die aus Sauerstoff, Stickstoff und Schwefel ausgewählt sind, und welches Ringsystem schließlich, sofern möglich, einen, zwei oder drei fakultative Substituenten tragen kann, die aus Fluor, Chlor, Brom, 1—6C-Alkyl, 1—6C-Alkoxy, 1—6C-Alkylthio, Trifluoromethyl, Hydroxy, 6—10C-Aryl, 7—11C-Arylalkyl, Carboxy, 2—6C-Carboxyalkyl, 2—6C-Alkoxycarbonyl, 3—10C-Alkoxycarbonylalkyl, 1—6C-Hydroxyalkyl, Heteroaryl(1—6C)alkyl, Furyl-, Thienyl, Pyrrolyl, Thiazolyl, Oxazolyl, Imidazolyl, Thiadiazolyl,

Oxadiazolyl, Triazolyl, Pyrazolyl, und Pyrimidyl ausgewählt sind, wobei, wenn die miteinander verbundenen $R^8$ und $R^9$ durch Heteroarylalkyl substituiert sind, das Heteroaryl ein 5- oder 6-gliedriger heterocyclischer Ring ist, der 1, 2, 3 oder 4 Heteroatome enthält, die aus Sauerstoff, Stickstoff und Schwefel ausgewählt sind, wobei dieser Ring gegebenenfalls durch ein oder zwei Methyle substituiert ist, oder worin $R^8$ und $R^9$ miteinander verbunden sind, so daß sie zusammen mit der N—C = N -Kette, an welche sie geknüpft sind, einen Ring der Formel V

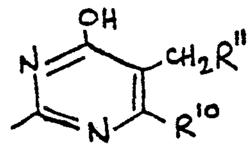

V

bilden, worin $R^{10}$ Wasserstoff oder 1—4C-Alkyl bedeutet und $R^{11}$ Furan-2-yl oder Thien-2-yl, das in der 5-Stellung substituiert ist, Phenyl, das in der 3- oder 4-Stellung substituiert ist, Pyrid-3-yl, das in der 5- oder 6-Stellung substituiert ist, Pyrid-4-yl, das in der 2-Stellung substituiert ist, oder Pyrid-2-yl, das in der 4- oder 5-Stellung substituiert ist, bedeutet, wobei der Substituent an $R^{11}$ die Formel

$$R^{12}R^{13}N{-}(CH_2)_m{-} \qquad VI$$

aufweist, worin $R^{12}$ und $R^{13}$ 1—4C-Alkyle sind oder $R^{12}$ und $R^{13}$ miteinander verbunden sind, so daß sie zusammen mit dem Stickstoff, an welchen sie geknüpft sind, einen Pyrrolidin-, Piperidin- oder Morpholinring bilden, und m 1 bis 4 ist;

sowie der pharmazeutisch zulässigen Säureadditionssalze davon, dadurch gekennzeichnet, daß ein Isoharnstoff der Formel VII oder VIII

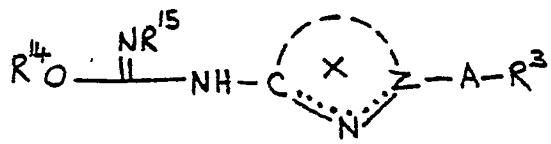

VII

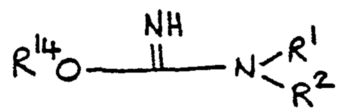

VIII

worin $R^{14}$ für gegebenenfalls substituiertes Aryl steht und $R^{15}$ für Wasserstoff oder einen der oben für $R^1$ angegebenen Werte außer Wasserstoff steht, mit Ammoniak oder einem Amin der Formel $R^1R^2NH$ bzw. mit einem Amin der Formel X

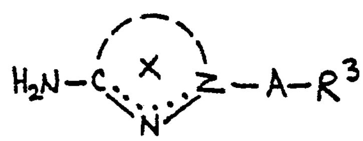

X

umgesetzt wird, worauf, wenn das Verfahren die Verbindung der Formel I in Form der freien Base liefert und ein Säureadditionssalz gewünscht wird, die Verbindung der Formel I in der freien Basenform mit einer Säure umgesetzt wird, die ein pharmazeutisch zulässiges Anion liefert.

2. Verfahren nach Anspruch 1, wobei $R^{14}$ für Phenyl oder Naphthyl steht, das gegebenenfalls durch ein oder zwei Substituenten substituiert ist, die aus Halogen, Nitro (nur eines), 1—6C-Alkyl, 1—6C-Alkoxy und Phenoxy ausgewählt sind.

3. Verfahren nach Anspruch 1 oder 2, welches in einem Verdünnungs- oder Lösungsmittel ausgeführt wird und welches durch die Anwendung von Wärme beschleunigt oder zu Ende geführt wird.

4. Verfahren nach einem der Ansprüche 1 bis 3, bei welchem in den Ausgangsmaterialien $R^1$ für 2,2,2-Trifluoroethyl steht, $R^2$ für Wasserstoff steht, der Ring X ein Pyrazolring ist, A für Tetramethylen steht und $R^3$ die Formel III aufweist, worin $R^6$ und $R^7$ Wasserstoff bedeuten.

**Revendications**

1. Procédé de production d'un dérivé de guanidine de formule I:

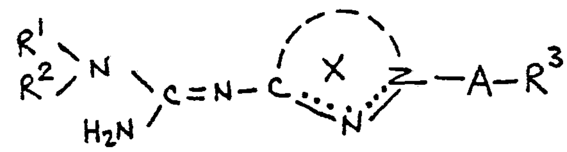

I

dans laquelle $R^1$ et $R^2$, qui peuvent être identiques ou différents, représentent de l'hydrogène ou un groupe alkyle en $C_1$ à $C_{10}$ ramifié ou non ramifié, cycloalkyle en $C_3$ à $C_8$ ou cycloalkylalkyle en $C_4$ à $C_{14}$, chaque groupe alkyle, cycloalkyle ou cycloalkylalkyle étant facultativement substitué par un ou plusieurs atomes d'halogènes choisis entre fluor, chlore et brome, sous réserve qu'au moins l'un de $R^1$ et $R^2$ soit un groupe alkyle, cycloalkyle ou cycloalkylalkyle substitué par un halogène et sous réserve qu'il n'y ait pas de substituant halogèno sur l'atome de carbone du groupe alkyle, cycloalkyle ou cycloalkylalkyle qui est directement attaché à l'azote,

ou bien —$R^2$ est l'hydrogène et —$R^1$ répond à la formule

$$R^4{-}E{-}W{-} \qquad \text{II}$$

dans laquelle W est une chaîne alkylénique en $C_2$ à $C_6$ non ramifiée qui est facultativement substituée par un ou deux radicaux alkyle en $C_1$ à $C_4$, E est l'oxygène, le soufre, un groupe sulfinyle, sulfonyle ou bien, lorsque $R^4$ est un reste alkyle, un groupe de formule $NR^5$ dans laquelle $R^5$ est un radical alkyle en $C_1$ à $C_6$, $R^4$ est l'hydrogène ou un reste alkyle en $C_1$ à $C_6$ non ramifié qui est facultativement substitué par un ou deux radicaux alkyle en $C_1$ à $C_4$, ou bien $R^4$ et $R^5$ forment conjointement avec l'atome d'azote auquel ils sont liés un noyau de pyrrolidine, pipéridine, morpholine, pipérazine ou N-Méthylpipérazine;

dans le noyau X, la ligne en pointillé est une double liaison d'un côté de l'azote et Z est un atome de carbone ou d'azote, en sorte que le noyau X est un noyau hétérocyclique aromatique pentagonal ou hexagonal qui contient au moins un atome d'azote et qui peut éventuellement contenir un ou deux autres hétéroatomes choisis entre oxygène, azote et soufre, ce noyau hétérocyclique pouvant, lorsque cela est possible, porter un ou ou deux substituants facultatifs, les substituants facultatifs du noyau X étant choisis entre le fluor, le chlore, le brome, un groupe alkyle en $C_1$ à $C_6$, alkoxy en $C_1$ à $C_6$, trifluorométhyle et hydroxy;

A est un groupe phénylène ou un groupe cycloalkylène en $C_5$ à $C_7$ ou une chaîne alkylénique en $C_1$ à $C_8$ qui est éventuellement substituée par un ou deux radicaux alkyle en $C_1$ à $C_3$ et dans laquelle sont éventuellement insérés, commùe partie du squelette de la chaîne, un ou deux groupes choisis entre de l'oxygène, du soufre, des groupes NH, alkyle en $C_1$ à $C_6$, *cis* et *trans*-vinyl ne, éthynylène, phénylène et cycloalkylène en $C_5$ à $C_7$, sous réserve que la jonction la plus courte entre le noyau X et $R^3$ ait au moins 3 atomes de carbone, que lorsqu'une insertion facultative est faite dans la chaîne A de telle sorte que le groupe inséré se trouve directement attaché à $R^3$, le groupe inséré soit autre chose que de l'oxygène, du soufre, un groupe NH ou N-alkyle et sous réserve que deux insertions choisies entre oxygène, soufre, NH et N-alkyle ne soient pas directement attachées l'une à l'autre;

—$R^3$ répond à la formule III:

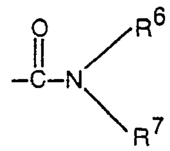

III

dans laquelle $R^6$ est l'hydrogène, un groupe hydroxy, alcanoylamino en $C_1$ à $C_6$, alkyle en $C_1$ à $C_6$, cycloalkyle en $C_3$ à $C_8$, cycloalkylalkyle en $C_4$ à $C_{12}$, alcényle en $C_2$ à $C_6$, alcynyle en $C_2$ à $C_6$, alkoxy en $C_1$ à $C_6$, hydroxyalkyle en $C_1$ à $C_6$, alkoxyaminoalkyle en $C_2$ à $C_{10}$, alkylthioalkyle en $C_2$ à $C_{10}$, dialkylaminoalkyle en $C_3$ à $C_{12}$, alcanoylaminoalkyle en $C_2$ à $C_8$, aroylaminoalkyle en $C_8$ à $C_{14}$, alkoxycarbonylalkyle en $C_3$ à $C_{10}$, carbamoylalkyle en $C_2$ à $C_8$, aryle en $C_6$ à $C_{10}$, arylalkyle en $C_7$ à $C_{11}$, hétéro-aryle ou hétéro-arylalkyle dont la partie hétéroaryle est un noyau aromatique hétérocyclique contenant un, deux ou trois hétéro-atomes choisis entre oxygène, azote et soufre, la partie alkyle du radical hétéroarylalkyle ayant 1 à 6 atomes de carbone et lorsque $R^6$ est ou contient un noyau aryle ou hétéro-aryle, ce noyau est facultativement substitué par un ou deux groupes choisis entre fluor, chlore, brome et iode, alkyle en $C_1$ à $C_6$, alkoxy en $C_1$ à $C_6$, alkylthio en $C_1$ à $C_6$, dialkylamino en $C_2$ à $C_6$, alcanoyle en $C_2$ à $C_6$, trifluorométhyle et hydroxy;

$R^7$ est l'hydrogène ou bien $R^6$ et $R^7$ s'associent pour former, conjointement avec l'azote auquel ils sont liés, un noyau saturé pentagonal, hexagonal ou heptagonal qui contient facultativement une double liaison et un atome additionnel d'oxygène ou un groupe N-alkyle en $C_1$ à $C_6$;

ou bien —$R^3$ répond à la formule IV:

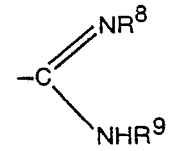

IV

dans laquelle $R^8$ et $R^9$ s'associent pour former, conjointement avec la chaîne N—C = N à laquelle ils sont liés, un système hétérocyclique monocyclique ou bicyclique composé de noyaux pentagonaux et/ou hexagonaux, ce système cyclique pouvant être partiellement insaturé ou totalement insaturé, pouvant facultativement comprendre d'autres hétéro-atomes choisis entre oxygène, azote et soufre et pouvant, lorsque cela est possible, porter un, deux un trois substituants facultatifs choisis entre du fluor, du chlore, du brome, un groupe alkyle en $C_1$ à $C_6$, alkoxy en $C_1$ à $C_6$, alkylthio en $C_1$ à $C_6$, trifluorométhyle, hydroxy, aryle en $C_6$ à $C_{10}$, arylalkyle en $C_7$ à $C_{11}$, carboxy, carboxyalkyle en $C_2$ à $C_6$, alkoxycarbonyle en $C_2$ à $C_6$, alkoxycarbonylalkyle en $C_3$ à $C_{10}$, hydroxyalkyle en $C_1$ à $C_6$, hétéroaryl-(alkyle en $C_1$ à $C_6$), furyle, thiényle, pyrrolyle, thiazolyle, oxazolyle, imidazolyle, thiadiazolyle, oxadiazolyle, triazolyle, pyrazolyle et pyrimidyle; et lorsque $R^8$ et $R^9$, en association, sont substitués par un groupe hétéro-arylalkyle, la partie hétéro-aryle est un noyau hétérocyclique pentagonal ou hexagonal qui contient 1, 2, 3 ou 4 hétéroatomes choisis entre oxygène, azote et soufre, ce noyau étant facultativement substitué par un ou deux groupes méthyle;

ou bien $R^8$ et $R^9$ s'associent pour former, conjointement avec la chaîne N—C = N à laquelle ils sont liés, un noyau de formula V:

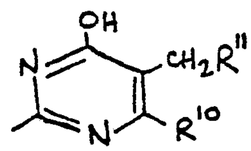

V

dans laquelle $R^{10}$ est l'hydrogène ou un groupe alkyle en $C_1$ à $C_4$ et $R^{11}$ est un groupe furanne-2-yle ou thién-2-yle substitué en position 5, phényle substitué en position 3 ou 4, pyrid-3-yle substitué en position 5 ou 6, pyrid-4-yle substitué en position 2 ou pyrid-2-yle substitué en position 4 ou 5, le substituant de $R^{11}$ répondant à la formule

$$R^{12}R^{13}N-(CH_2)_m- \qquad VI$$

dans laquelle $R^{12}$ et $R^{13}$ sont des groupes alkyle en $C_1$ à $C_4$, ou bien $R^{12}$ et $R^{13}$ s'associent pour former, conjointement avec l'azote auquel ils sont liés, un noyau de pyrrolidine, pipéridine ou morpholine et *m* a une valeur de 1 à 4;

et de ses sels d'addition d'acides pharmaceutiquement acceptables, caractérisé par la réaction d'une isourée de formule VII ou VIII:

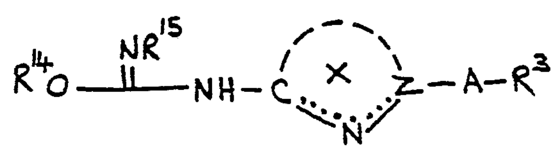

VII

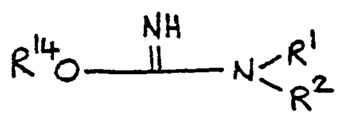

VIII

dans laquelle $R^{14}$ est un groupe aryle éventuellement substitué et $R^{15}$ est l'hydrogène ou a l'une des valeurs données ci-dessus pour $R^1$ autres que l'hydrogène, avec l'ammoniac ou une amine qui répond à la formule $R^1R^2NH$ ou avec une amine de formule X:

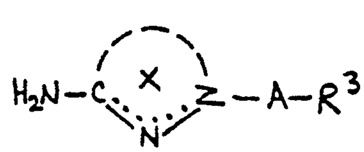

X

respectivement;

après quoi, lorsque le procédé donne le composé de formule I sous la forme de la base libre et que l'on

désire obtenir un sel d'addition d'acidé, le composé de formule I sous la forme de la base libre est amené à réagir avec un acide qui apporte un anion pharmaceutiquement acceptable.

2. Procédé suivant la revendication 1, dans lequel $R^{14}$ est un groupe phényle ou napthyle qui est facultativement substitué par un ou deux substituants choisis entre halogène, nitro (un seul), alkyle en $C_1$ à $C_6$, alkoxy en $C_1$ à $C_6$ et phénoxy.

3. Procédé suivant la revendication 1 ou 2, qui est mis en oeuvre dans un diluant ou un solvant et qui peut être accéléré ou achevé par l'utilisation de la chaleur.

4. Procédé suivant l'une quelconque des revendications 1 à 3, dans lequel, dans les matières de départ, $R^1$ est un groupe 2,2,2-trifluoroéthyle, $R^2$ est l'hydrogène, le noyau X est un noyau de pyrazole, A est un groupe tétraméthylene et $R^3$ répond à la formule III dans laquelle $R^6$ et $R^7$ sont de l'hydrogène.